(19) Europäisches Patentamt
European Patent Office
Office européen des brevets

(11) **EP 0 942 973 B1**

(12) **FASCICULE DE BREVET EUROPEEN**

(45) Date de publication et mention
de la délivrance du brevet:
**15.03.2006 Bulletin 2006/11**

(51) Int Cl.:
*C12N 15/12* (2006.01)    *C07K 14/705* (2006.01)
*A61K 38/17* (2006.01)    *C12N 15/62* (2006.01)
*C07K 16/46* (2006.01)    *C12N 1/21* (2006.01)
*C12N 5/10* (2006.01)    *C12N 1/21* (2006.01)
*C12N 1/19* (2006.01)

(21) Numéro de dépôt: **97947136.4**

(22) Date de dépôt: **25.11.1997**

(86) Numéro de dépôt international:
**PCT/FR1997/002126**

(87) Numéro de publication internationale:
**WO 1998/023741 (04.06.1998 Gazette 1998/22)**

(54) **MUTANTS DE LA PROTEINE LAG-3, LEUR EXPRESSION ET UTILISATION**

MUTANTEN DES LAG-3 PROTEINS, IHRE EXPRESSION UND VERWENDUNG

MUTANTS OF THE LAG-3 PROTEINS, PRODUCTS FOR THE EXPRESSION OF THESE MUTANTS AND USE

(84) Etats contractants désignés:
**AT BE CH DE DK ES FI FR GB GR IE IT LI LU MC NL PT SE**
Etats d'extension désignés:
**AL LT LV MK RO SI**

(30) Priorité: **28.11.1996 FR 9614608**

(43) Date de publication de la demande:
**22.09.1999 Bulletin 1999/38**

(73) Titulaires:
• **Institut Gustave Roussy**
**94805 Villejuif Cédex (FR)**
• **INSTITUT NATIONAL DE LA SANTE ET DE LA RECHERCHE MEDICALE (INSERM)**
**75013 Paris (FR)**
• **Applied Research Systems ARS Holding N.V. Curacao (AN)**

(72) Inventeurs:
• **EL TAYAR, Nabil**
**Milton, MA 02186 (US)**
• **MASTRANGELI, Renato**
**I-00146 Rome (IT)**
• **HUARD, Bertrand**
**F-94240 L'Hay les Roses (FR)**
• **TRIEBEL, Frédéric**
**F-78000 Versailles (FR)**

(74) Mandataire: **Breese, Pierre et al**
**BREESE DERAMBURE MAJEROWICZ**
**38, avenue de l'Opéra**
**75002 Paris (FR)**

(56) Documents cités:
**WO-A-95/30750**

• **TRIEBEL F. ET AL.: "LAG-3, a novel lymphocyte activation gene closely related to CD4." J. EXP. MED., vol. 171, 1990, pages 1393-1405, XP000672284 cité dans la demande**
• **BAIXERAS E. ET AL.: "Characterization of the lymphocyte activation gene 3-encoded protein. A new ligand for human leukocyte antigen class II antigens." J. EXP. MED., vol. 176, 1992, pages 327-337, XP000672285 cité dans la demande**
• **HUARD B ET AL: "CD4/MAJOR HISTOCOMPATIBILITY COMPLEX CLASS II INTERACTION ANALYZED WITH CD4- AND LYMPHOCYTE ACTIVATION GENE-3 (LAG-3)-IG FUSION PROTEINS" EUROPEAN JOURNAL OF IMMUNOLOGY, vol. 25, no. 9, septembre 1994, pages 2718-2721, XP000672291 cité dans la demande**
• **Huard B. et al.: 'The lymphocyte activa- tion gene product LAG-3 is a ligand for class II antigens.' in: ABSTRACTS FROM THE 'PROCEEDINGS OF THE INTERNATIONAL CONGRESS OF IMMUNOLOGY', Budapest, Congress 8; page 281, A19; DE, Berlin, Springer, 1992; XP002037068**

EP 0 942 973 B1

• FLEURY S. ET AL.: "Mutational analysis of the interaction between CD4 and class II MHC: Class II antigens contact CD4 on a surface opposite the gp120-binding site." CELL, vol. 66, 1991, pages 1037-1049, XP002037066

• HUARD B. ET AL.: "Characterization of the major histocompatibility complex class II binding site on LAG-3 protein." PROC. NATL. ACAD. SCI. USA, vol. 94, no. 11, mai 1997, pages 5744-5749, XP002037067

**Description**

L'invention concerne de nouveaux polypeptides correspondant à des formes mutantes de la protéine LAG-3 soluble ou de ses fragments.

Le gène d'activation des lymphocytes 3 (LAG-3) exprimé chez les cellules T activées et les cellules NK humaines code pour une protéine membranaire de type I de 498 acides aminés avec quatre domaines extra cellulaires de la superfamille des immunoglobulines (IgSF) (1). L'analyse de sa séquence a révélé de nombreuses identités avec des séries de séquences en acides aminés présentes à des positions correspondantes sur le récepteur CD4, bien que l'homologie de séquences globale entre ces deux molécules soit à peine au-dessus d'un niveau de base (approximativement 20 % d'identités de séquences). I1 existe aussi des homologies de séquences internes dans la molécule LAG-3, entre les domaines 1 (D1) et 3 (D3) ainsi qu'entre les domaines 2 (D2) et 4 (D4), ce qui suggère que LAG-3 a évolué, comme CD4, par une duplication génique à partir d'une structure préexistante composée de deux IgSF (1). LAG-3 et CD4 peuvent donc être considérés comme des "premiers cousins" dans l'évolution à l'intérieur de la famille des IgSF (2).

Les auteurs de la présente invention ont montré dans des travaux antérieurs, en utilisant un test d'adhésion cellulaire quantitatif, que la formation de rosettes entre des cellules COS-7 transfectées par LAG-3 et des lymphocytes B exprimant les molécules du complexe majeur d'histocompatibilité de classe II (CMH) est spécifiquement dépendante de l'interaction entre LAG-3 et les molécules de classe II du CMH (2). Une liaison directe et spécifique de LAG-3 à différentes molécules de classe II humaines (y compris différents allèles et isotypes) ainsi qu'à des molécules de classe II murines et de singe a été également observée avec une protéine de fusion LAG-3Ig (3). Cette globuline recombinante dimérique LAG-3Ig se lie au résidu monomorphe des molécules de classe II du CMH avec une plus forte affinité ($K_d$ = 60 nM à 37°C) que le CD4-Ig (4) ; LAG-3Ig est en fait capable de bloquer l'interaction molécule de classe II/CD4 dans un test d'adhésion intercellulaire (4).

Le rôle de l'interaction LAG-3/molécule de classe II a été étudié en utilisant des anticorps monoclonaux spécifiques de LAG-3 (5) et des molécules. LAG-3Ig (6). Cette interaction conduit à une régulation négative de l'activation de clones de cellules T. L'obtention d'une interaction LAG-3/molécule de classe II est induite par des contacts entre cellules T, probablement via un signal négatif des molécules de classe II dans la cellule T.

De façon générale, LAG-3 est exprimé uniquement après l'activation lymphocytaire aussi bien *in vivo* qu'*in vitro* (5) et donc ne joue pas un rôle dans la phase d'induction de la réponse, contrairement au CD4. Par ailleurs, des expériences de blocage avec des anticorps monoclonaux ont montré que LAG-3 ne participe pas à la phase de reconnaissance par des clones de cellules T CD4 restreints par les molécules de classe II. Le rôle fonctionnel de LAG-3 est donc fortement différent de celui des autres ligands des molécules du CMH, CD4 et CD8.

Dans la demande PCT/FR95/00593, les auteurs de la présente invention ont montré que certaines fractions polypeptidiques solubles de LAG-3 étaient également capables de se lier aux molécules de classe II. Ceux-ci ont également souligné l'importance potentielle des acides aminés de la région comprise entre le résidu 46 et le résidu 77, et notamment les positions 73, 75, 76 et 77.

Les auteurs de l'invention ont à présent cherché à caractériser de façon précise la/les région(s) de LAG-3 spécifiquement impliquées dans la liaison aux molécules de classe II, et, pour ce faire, ont synthétisé plusieurs formes mutées de LAG-3, possédant des mutations ciblées dans les deux premiers domaines de la région extracytoplasmique de la protéine mature, c'est-à-dire la protéine dépourvue de peptide signal, de séquence N-terminale L-Q-P-G-A-E. Le domaine D1 s'étend de l'acide aminé N° 1 (L), l'acide aminé N° 149 (M) ; le domaine D2 s'étend de l'acide aminé N° 150 (T) à 239 (G). De façon surprenante, les auteurs de l'invention ont montré que la substitution d'un seul acide aminé pouvait induire des modifications notoires dans l'affinité de LAG-3 pour les molécules de classe II, soit en augmentant fortement la liaison de LAG-3 à ces protéines, soit au contraire en l'inhibant partiellement ou en totalité.

La présente invention a donc pour objet un polypeptide purifié correspondant à une forme mutée de la protéine LAG-3 soluble ou d'un de ses fragments comprenant le domaine extracellulaire D1 et D2 consistant :

- soit en une substitution d'acide aminé à l'une des positions choisie parmi le groupe constitué par :

 la position 73 où l'arginine est substituée par l'acide glutamique (R73E),
 la position 75 où l'arginine est substituée par l'alanine (R75A) ou par l'acide glutamique (R75E),
 la position 76 où l'arginine est substituée par l'acide glutamique (R76E),

 ou en une combinaison de deux ou trois des substitutions précédentes,

- soit en une substitution d'acide aminé à l'une des positions choisie parmi le groupe constitué par :

 la position 30 où l'acide aspartique est substitué par l'alanine (D30A),
 la position 56 où l'histidine est substitué, par l'alanine (H56A),

la position 77 où la tyrosine est substituée par la phénylalanine (Y77F),
la position 88 où l'arginine est substituée par l'alanine (R88A),
la position 103 où l'arginine est substituée par l'alanine (R103A),
la position 109 où l'acide aspartique est substitué par l'acide glutamique (D109E),
la position 115 où l'arginine est substituée par l'alanine (R115A) ;

ou en une délétion de la région comprise entre la position 54 (P) et la position 66 (A).

Elle a également pour objet l'utilisation de ces mutants de LAG-3 soluble pour la fabrication de médicaments, notamment des médicaments peptidomimétiques de la molécule LAG-3 c'est-à-dire se fixant de façon similaire sur les molécules du CMH de classe II et ayant le même type d'activité immunosuppressive que la molécule LAG-3.

L'invention vise également des compositions pharmaceutiques comprenant à titre de principe actif l'une des formes mutées de la protéine LAG-3 définie précédemment.

Dans ce qui suit, les polypeptides définis ci-dessus seront dénommés "mutants de LAG-3".

Cette définition vise les polypeptides correspondant à des formes mutées de la protéine LAG-3 soluble entière ou de fragments de LAG-3 comprenant les domaines extracellulaires D1 et D2, notamment le fragment composé des deux domaines D1 et D2.

La protéine LAG-3 soluble correspond à la séquence comprise entre le premier résidu N-terminal et le résidu 412 de la protéine mature (dépourvue de peptide signal).

Selon un premier mode de réalisation, l'invention a pour objet des polypeptides purifiés mutants de LAG-3 soluble ayant une affinité de liaison aux molécules de classe II du CMH supérieure à l'affinité de la protéine LAG-3 sauvage. Les mutations induites dans ces peptides sont préférentiellement localisées au niveau de la boucle externe de LAG-3, localisée entre le résidu 46 et le résidu 77.

Plus précisément, l'invention vise un polypeptide purifié correspondant à une forme mutée de la protéine LAG-3 soluble ou d'un de ses fragments comprenant les deux domaines extracellulaires D1 et D2, consistant en une substitution d'acide aminé à l'une des positions choisie parmi le groupe constitué par :

la position 73 où l'arginine est substituée par l'acide glutamique (R73E),
la position 75 où l'arginine est substituée par l'alanine (R75A) ou par l'acide glutamique (R75E),
la position 76 où l'arginine est substituée par l'acide glutamique (R76E),

ou en une combinaison de deux ou trois des substitutions précédentes.

Préférentiellement, les mutants de LAG-3 résultent d'une combinaison de deux ou trois substitutions choisies parmi le groupe constitué par :

(R73E + R75A),
(R73E + R76E),
(R75A + R76E),
(R73E + R75A + R76E).

Ces différents mutants seront dénommés ci-après "mutants positifs".

Selon un autre mode de réalisation, l'invention a pour objet un polypeptide purifié correspondant à une forme mutée de la protéine LAG-3 soluble ou d'un de ses fragments comprenant le domaine extracellulaires D1 et D2, consistant en une substitution d'acide aminé à l'une des positions choisie parmi le groupe constitué par :

la position 30 où l'acide aspartique est substitué par l'alanine (D30A),
la position 56 où l'histidine est substitué par l'alanine (H56A),
la position 77 où la tyrosine est substituée par la phénylalanine (Y77F),
la position 88 où l'arginine est substituée par l'alanine (R88A),
la position 103 où l'arginine est substituée par l'alanine (R103A),
la position 109 où l'acide aspartique est substitué par l'acide glutamique (D109E),
la position 115 où l'arginine est substituée par l'alanine (R115A) ;

ou en une délétion de la région comprise entre la position 54 (P) et la position 66 (A).

Ces mutants seront dénommés ci-après "mutants négatifs".

Les mutants ponctuels de LAG-3 selon la présente invention peuvent être obtenus par des méthodes de mutagénèse dirigée.

La méthode de mutagénèse dirigée, qui permet de créer à volonté des mutations définies à la fois par leur nature et par leur position est aujourd'hui bien connue de l'homme de l'art. Son principe repose sur une hybridation d'un oligonucléotide amorce portant la mutation désirée sur une matrice d'ADN dénaturée. Après, formation d'un duplex entre le brin d'ADN muté produit par élongation de l'amorce et le brin d'ADN sauvage, des cycles successifs de réplication dans des conditions appropriées permettent de produire de l'ADN muté sur les deux brins.

On peut considérer aujourd'hui que la mutagénèse dirigée recouvre un ensemble de protocoles et de techniques que l'homme du métier pourra sélectionner ou modifier en fonction de ses besoins. Schématiquement, la mutagénèse dirigée peut être réalisée selon trois types d'approches, qui, dans un ordre chronologique, sont : la technologie du simple brin, la technologie double brin et la technologie par PCR ; chacune de ces trois approches possédant plusieurs variantes plus ou moins adaptées aux objectifs et contraintes spécifiques de l'expérimentateur.

Dans le cadre de la présente invention, la mutagénèse dirigée est réalisée sur de l'ADN codant pour la protéine LAG-3 soluble ou un de ses fragments. Les séquences nucléotidiques isolées codant pour les différents mutants de LAG-3, ainsi que leurs séquences nucléotidiques complémentaires, sont un autre aspect de l'invention.

Les polypeptides mutants de LAG-3, résultant des mutations induites sur le gène de LAG-3, sont obtenus par des techniques de production de produits recombinants après introduction de leur séquence nucléotidique codante dans un hôte cellulaire approprié.

Dans ce cas, la séquence nucléotidique utilisée est placée sous le contrôle de signaux permettant son expression dans l'hôte choisi. L'hôte cellulaire utilisé peut être choisi parmi des systèmes procaryotes, comme les bactéries, ou eucaryotes, comme par exemple les levures, cellules d'insectes, CHO (cellules d'ovaires de hamster chinois) ou tout autre système avantageusement disponible dans le commerce. Un hôte cellulaire préféré pour l'expression des polypeptides de l'invention est constitué par la lignée fibroblastique COS-7.

Le vecteur doit comporter un promoteur, des signaux d'initiation et de terminaison de la ; traduction, ainsi que les régions appropriées de régulation de la transcription. Il doit pouvoir être maintenu de façon stable dans la cellule et peut éventuellement posséder des signaux particuliers spécifiant la sécrétion de la protéine traduite.

Ces différents signaux de contrôle sont choisis en fonction de l'hôte cellulaire utilisé. A cet effet, les séquences nucléotidiques codant pour les peptides selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation.

Les vecteurs d'expression des polypeptides des mutants de LAG-3 définis ci-dessus font également partie de la présente invention.

Dans le cas des cellules COS-7, la transfection peut être réalisée à partir du vecteur PCDM8, comme décrit dans(2).

L'invention vise en outre les cellules transfectées par ces vecteurs d'expression. Ces cellules peuvent être obtenues par l'introduction dans des cellules hôtes d'un vecteur d'expression d'un mutant de LAG-3 tel que défini ci-dessus, puis la mise en culture desdites cellules dans des conditions permettant la réplication et/ou l'expression de la séquence nucléotidique transfectée codant pour un mutant de LAG-3.

Lorsque les mutants de LAG-3 sont exprimés à la surface des cellules transfectées, celles-ci peuvent être utilisées dans des tests de liaison et d'adhésion cellulaire pour étudier la capacité de ces mutants à se lier à différents ligands, notamment des antigènes membranaires de certaines populations cellulaires, en particulier les molécules du CMH de classe II.

Les cellules hôtes sont également utilisables; dans une méthode de production d'un polypeptide mutant de LAG-3, méthode elle-même comprise dans la présente invention, et caractérisée en ce que l'on cultive les cellules transfectées dans des conditions permettant l'expression d'un polypeptide recombinant mutant de LAG-3, et que l'on récupère ledit polypeptide recombinant.

Les procédés de purification utilisés sont connus de l'homme du métier. Le polypeptide recombinant peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps mono ou poZyclonaux spécifiques, etc.

Une variante avantageuse consiste à produire un mutant de LAG-3 recombinant fusionné à une protéine "porteuse", par exemple une immunoglobuline ou une région d'immunoglobuline, pour former une protéine chimère. L'un des avantages de ce système est qu'il permet une stabilisation et une diminution de la protéolyse du produit recombinant et une simplification de la purification lorsque le partenaire de fusion possède une affinité pour un ligand spécifique.

Des protéines de fusion constituées de fragments du domaine extracytoplasmique de LAG-3 liés à la région jonction -$CH_2$-$CH_3$ de la chaîne lourde d'une immunoglobuline humaine (Ig) ont notamment été décrites dans la demande PCT/FR95/00593.

Parmi les peptides mutants de LAG-3 selon l'invention, les mutants positifs, c'est-à-dire ceux présentant une affinité pour les molécules du CMH de classe II plus forte que la molécule sauvage, sont avantageusement utilisables pour la fabrication de compositions pharmaceutiques à activité immunomodulatrice.

Sont également comprises dans l'invention lesdites compositions pharmaceutiques, comprenant à titre de principe actif un mutant de LAG-3, associé à un véhicule pharmaceutiquement acceptable. De telles compositions offrent une nouvelle approche pour le contrôle des réponses immunitaires impliquant une interaction cellulaire entre cellules T activées et cellules exprimant les molécules du CMH de classe II. Ces compositions sont par exemple utiles pour exercer un effet immunosuppresseur dans le cas de pathologies liées à des réactions immunologiques anormales ou exacerbées telles que des maladies auto-immunes, ou le rejet de greffes d'organes.

Les compositions pharmaceutiques de l'invention sont également utiles pour prévenir ou ralentir la croissance tumorale IN VIVO. L'immunodépression cellulaire T naturelle au sein des tumeurs humaines est une réalité biologique maintenant reconnue. Aussi, de petites molécules intervenant dans l'interaction LAG-3/molécules du CMH de classe II, offrent-elles une nouvelle voie d'intervention en immmunothérapie anti-cancéreuse sur ce type de relations entre une tumeur et l'organisme hôte.

Les compositions thérapeutiques selon la présente invention peuvent être formulées selon les techniques habituelles. Le véhicule peut être de forme variée en fonction de la voie d'administration choisie: orale, parentérale, sublinguale, rectale ou nasale.

Pour les compositions à administration parentérale, le véhicule comprendra généralement de l'eau stérile ainsi que d'autres ingrédients éventuels promouvant la solubilité ou l'aptitude à la conservation de la composition. Les voies d'administration parentérales peuvent consister en injections intraveineuses, intramusculaires ou soucutanées.

La composition thérapeutique peut être à libération prolongée, notamment pour les traitements à long terme, par exemple le traitement des maladies auto-immunes ou le contrôle du rejet de greffe d'organe. La dose à administrer dépend du sujet à traiter, notamment de la capacité de son système immunitaire à atteindre le degré de protection souhaité. Les quantités précises d'ingrédient actif à i administrer peuvent être déterminées sans effort par le praticien qui initiera le traitement.

Les compositions thérapeutiques selon l'invention peuvent comprendre en plus d'un ou , plusieurs mutants de LAG-3 un autre ingrédient actif, éventuellement lié par une liaison chimique au mutant de LAG-3. On citera à titre d'exemple des mutants de LAG-3 solubles selon l'invention fusionnés à une toxine: par exemple la ricine ou l'anatoxine diphtérique, susceptibles de se fixer à des molécules de CMH de classe II et de tuer les cellules cibles, par exemple des cellules leucémiques ou de mélanome, ou fusionnés à un radioisotope.

L'utilisation des peptides mutants négatifs, c'est-à-dire ceux dont l'affinité pour les molécules du CMH de classe II est réduite totalement ou partiellement, illustre un autre aspect avantageux de l'invention.

Les propriétés de ces peptides fournissent en effet des informations sur les interactions moléculaires entre LAG-3 et les molécules du CMH de classe II, ce qui les rend utiles pour la fabrication de molécules agonistes ou antagonistes de l'interaction entre LAG-3 et les molécules du CMH de classe II.

Comme cela sera illustré ci-après par les exemples, différentes structures de LAG-3 sont vraisemblablement responsables de l'oligomérisation et de l' interaction avec les molécules du CMH de classe II. Certains mutants négatifs de LAG-3 peuvent permettre une oligomérisation avec la protéine sauvage (sous forme soluble ou liée à la membrane), et modifier l'interaction des complexes résultants avec les molécules du CMH de classe II. De tels mutants peuvent ainsi induire une réversion de certaines fonctions de LAG-3. En conséquence, il apparaît que les mutants négatifs de LAG-3 constituent des outils intéressants pour développer des agonistes ou antagonistes de petite taille dérivés de LAG-3 ou des molécules du CMH de classe II.

D'autres caractéristiques et avantages de l'invention sont illustrés par les exemples dans la suite de la description, ainsi que par les figures dont les légendes sont indiquées ci-après.

## LEGENDE DES FIGURES

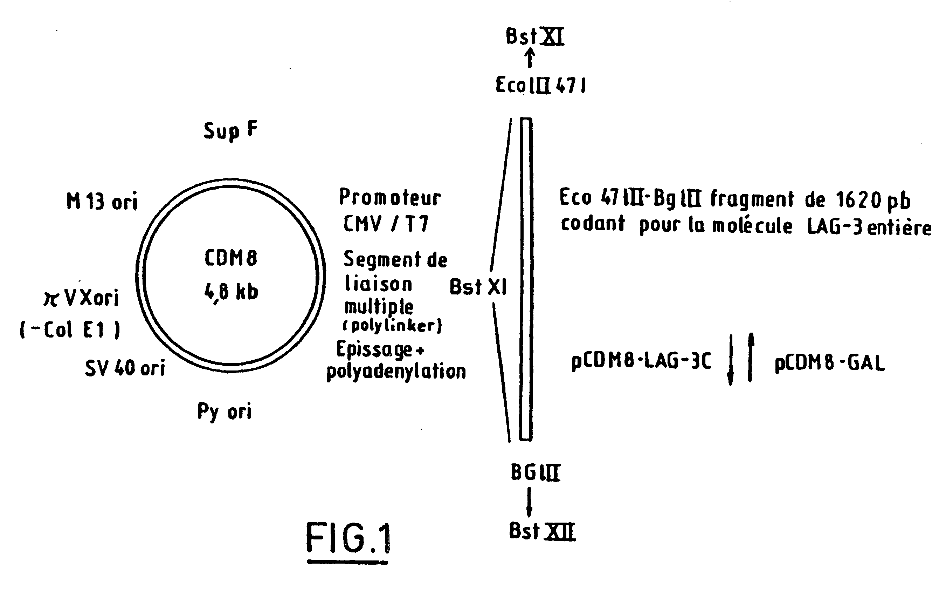

- La figure 1 représente le vecteur; d'expression pCDM8-LAG-3 et pCDM8-GAL. Le fragment FDC contenant le gène codant pour LAG-3 a été introduit dans le site BstXI de pCDM8 sous contrôle du promoteur du cytomégalovirus (pCDM8-LAG-3C) ou dans le sens inverse (pCDMB-GAL).
- La figure 2 représente les séquences nucléotidique et protéique du premier domaine D1 de LAG-3. La séquence nucléotidique du domaine D1 de LAG-3 est indiquée ligne 2. Les nucléotides sont positionnés (ligne 1) selon la numérotation des séquences LAG-3 déposées à l'EMBL (X51984). La séquence protéique est donnée en utilisant le code des acides aminés à une lettre (ligne 3). La position de chaque acide aminé est indiquée ligne 4. La séquence de l'extrémité N-terminale (Leu 1) a été déterminée par séquençage protéique de la molécule LAG-3 purifiée de la membrane de lymphocytes humains activés. La position des amorces utilisées pour amplifier les séquences à mutagénéiser est indiquée par les flèches. Les sites SfanI et EspI, utilisés pour digérer les fragments d'ADN sont soulignés. Les codons R73, R75 et R76 sont surmontés d'astérisques. Le fragment d'ADN de 103 paires de bases qui a été sous-cloné pour générer les formes mutées de LAG-3 est indiqué en caractères gras.

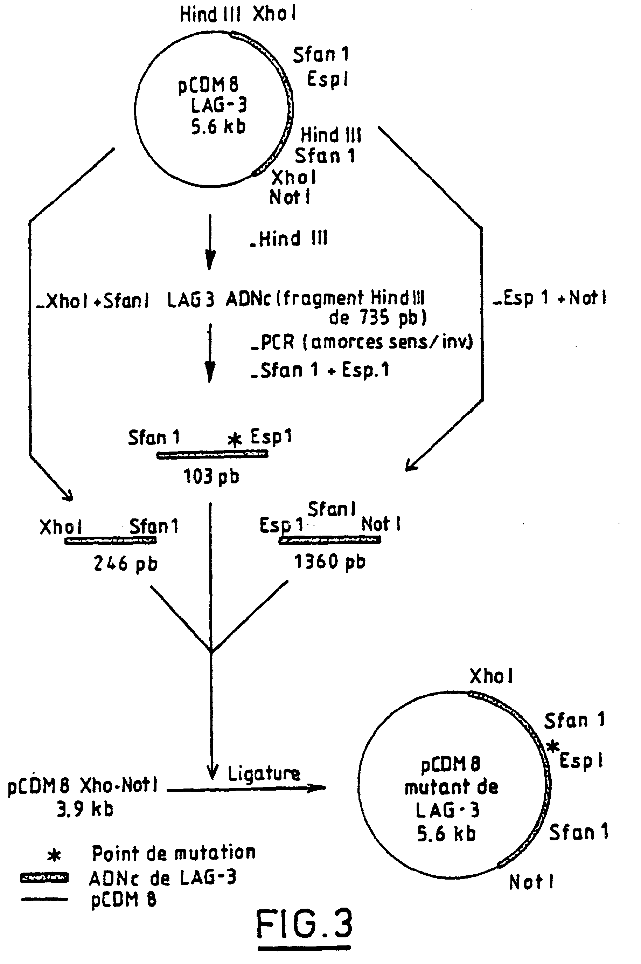

- La figure 3 représente une stratégie utilisée pour engendrer les formes mutées de LAG-3. Les vecteurs d'expression codant pour les formes mutées de LAG-3 ont été obtenus par liaison de trois sous-fragments de pCDM8-LAG-3C et d'un fragment SfanI-EspI obtenu par amplification par PCR en utilisant des amorces contenant les mutations ponctuelles appropriées.

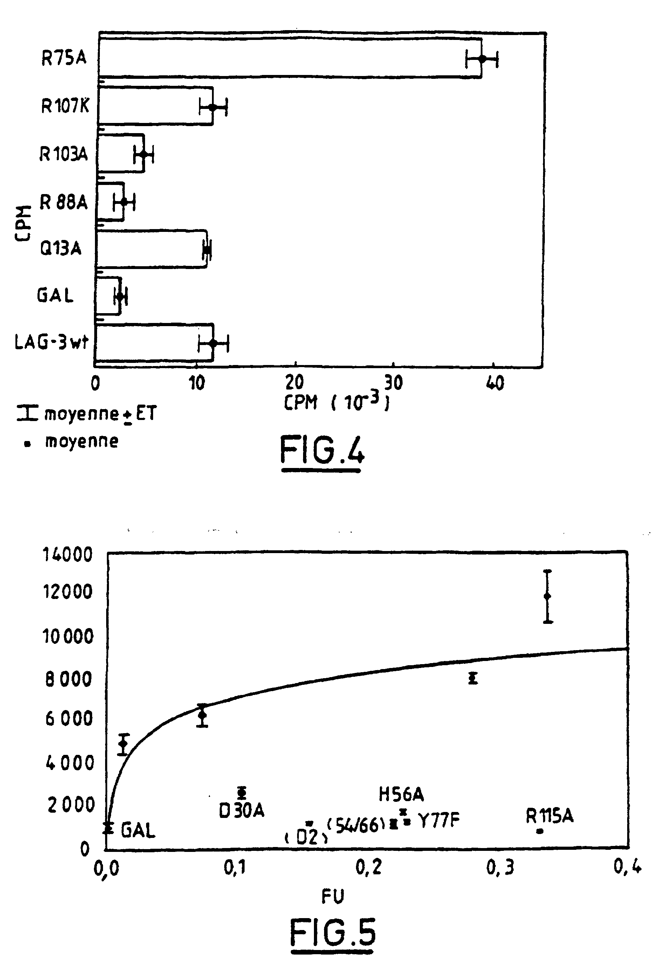

- La figure 4 représente la mesure quantificative de la liaison cellulaire entre des transfectants LAG-3 et des cellules B exprimant les molécules du CMH de classe II.

La liaison spécifique des cellules Daudi aux transfectants LAG3 sélectionnés a été quantifiée après marquage radioactif au $^{51}$Cr des cellules Daudi (valeurs obtenues dans des puits dupliqués).

Le taux de liaison non spécifique est indiqué par la liaison aux transfectants contenant le vecteur pCDM8 portant le gène LAG-3 inséré en orientation inverse (pCDM8-GAL).

- La figure 5 représente une comparaison de la liaison des mutants de LAG-3 aux molécules de classe II du CMH par rapport à celle de la molécule LAG-3 sauvage (wt) à différents taux d'expression.

Les cellules Cos-7 ont été respectivement transfectées avec GAL (0,3 $\mu$g), 0,03, 0,1, 0,3 et 1 $\mu$g d'ADN de pCDM8 LAG-3 sauvage, (symbole en cercle ouvert o). L'ADN de LAG-3 mutant a été utilisé à 0,03 $\mu$g par transfection. L'expression de LAG-3 est indiquée en unité de fluorescence arbitraire (FU) en utilisant 15A9, et la liaison aux molécules de classe II a été déterminée par comptage des coups par minute (cpm) dans un test d'adhésion cellulaire utilisant des cellules Daudi marquées au $^{51}$Cr.

- La figure 6 représente des vecteurs d'expression codant pour LAG-3D1D2Ig et LAG-3D1D4Ig. Les fragments d'ADN codant soit pour les 239 ou les 412 premiers acides aminés de LAG-3 ont été introduits à la place de la séquence codant pour la partie extracellulaire de CD8 dans pCDM7-CD8Ig.
- La figure 7 représente une expérience de liaison de LAG-3 aux cellules Daudi exprimant les molécules du CMH de classe II.

La liaison de CD8-Ig, LAG-3Ig (D1D4 ou D1D2, 100 nM chacun) est révélée par du sérum de chèvre anti-immunoglobulines humaines marqué au F-ITC. La liaison de l'anticorps monoclonal 9.49 (anti-DR) est révélée par du sérum de chèvre anti-immunoglobulines de souris marqué au F-ITC. L'OKT3 (anti-CD3) est un anticorps témoin IgG1 du même isotype.

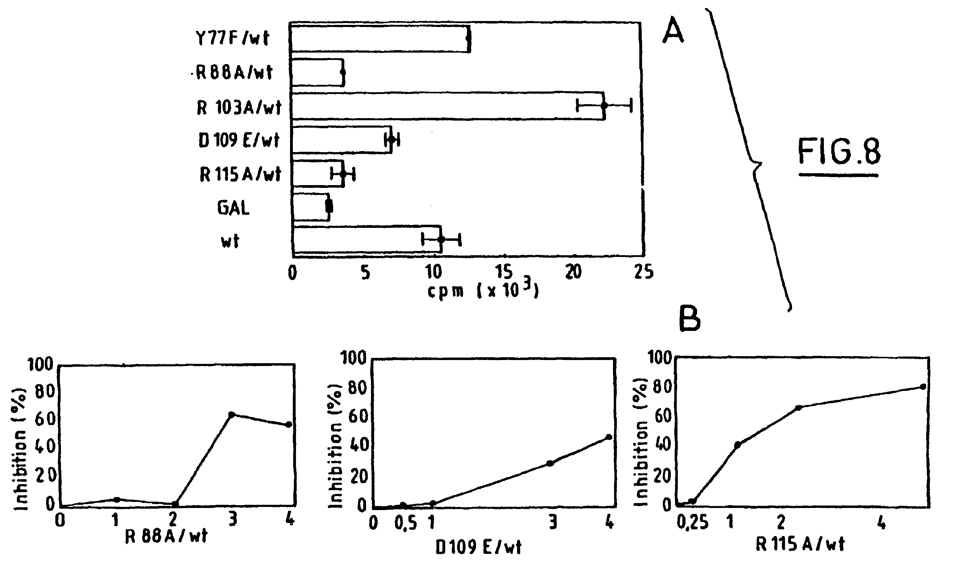

- La figure 8 représente une expérience montrant les effets d'une co-expression de certains mutants négatifs avec LAG-3 sauvage (wt) sur les interactions cellulaires.

Une co-expression des molécules de LAG-3 mutant R88A, D109E et R115A avec la protéine LAG-3 sauvage inhibe la liaison entre les cellules Cos transfectées et les cellules Daudi exprimant les molécules du CMH de classe II. Les quantités d'ADN utilisées pour la transfection étaient de 10 $\mu$g/ml pour LAG-3 sauvage (wt) et le contrôle négatif (GAL) et 40 $\mu$g/ml pour les mutants (figure 8a). La liaison aux molécules de classe II a été déterminée par comptage des coups par minute (cpm) dans un test d'adhésion cellulaire utilisant des cellules Daudi marquées au $^{51}$Cr. La figure 8b montre l'inhibition de la liaison des cellules Daudi en fonction de la quantité d'ADN transfecté codant pour R88A, D109E ou R115A. L'ADN de LAG-3 wt (10 $\mu$g/ml) a été mélangé à des quantités variables d'ADN des molécules mutantes (ADN mutant/ADN sauvage indiqué en abcisse) et de l'ADN de pCDM8 a été ajouté pour maintenir la quantité totale d'ADN transfecté à 50 $\mu$g/ml.

## MATERIALS ET METHODES

### . Anticorps monoclonaux et protéines de fusion

Les anticorps monoclonaux anti-LAG-3, 1784, 4F4 (spécifique de l'épitope LAG-3.1), 11E3 (LAG-3.2) et 15A9 (LAG3.3) (2), (5) ont été préalablement décrits. Les dimères LAG-3IgD1D4 et LAG-3IgD1D2 ont été préparés comme décrit précédemment (3). Des milieux conditionnés de cellules Cos-7 ont été utilisés en tant que source de protéines de fusion immunoglobuliniques (Ig) . Les surnageants sont purifiés sur colonne d'immunoaffinité ConA (fixant la région Fe des Ig humains).

### . Vecteurs d'expression des mutants de LAG-3 et tests d'adhésion

Le plasmide pCD8 est un vecteur d'expression développé par Seed (1987) permettant le clonage d'ADNc et leur expression soit par des bactéries soit par des cellules eucaryotes. Il contient une séquence ColE1 et SUP F (suppresseur d'ARNt) permettant l'expression de résistance à la tétracycline et à l'ampiciline chez des bactéries contenant un épisome P3 (Kan$^R$, amber tet$^R$, ambrer amp$^R$). Cet épisome confère aux souches bactériennes une résistance à la kanamycine. pCDM8 est composé d'un site de clonage multiple ("polylinker") de 358 paires de bases (pb), des séquences activatrices ("enhancer") du cytomégalovirus (CMV) et une origine de réplication des virus SV40 et Polyoma. La délétion de 800 paires de bases de pCDM8 contenant l'origine de réplication du virus Polyoma a permis d'engendrer le plasmide pCDM7.

La sélection des amorces a été réalisée à l'aide de recherche informatique (environnement "PC/GENE") sur des critères de sélection standards tels que : taille de l'amorce, Tm (température de fusion), richesse en GC, absence d'homologie floues.

Les mélanges réactionnels, incluant les , séquences plasmidiques de pCDM8 ou pCDM7, ont été utilisés pour transformer des bactéries *Escherichia coli* souche MC1061/P3. Cette souche de bactérie possède l'épisome P3 de 60 paires de bases en un petit nombre de copies, qui code pour une résistance naturelle à la kanamycine et pour un gène de type "amber" inductible, pour l'ampicilline et pour la tétracycline. Pour éviter des réversions spontanées, la sélection de colonies porteuses de plasmides recombinés a été réalisée sur des milieux contenant de l'ampicilline et de la tétracycline.

La production des vecteurs d'expression a été réalisée par culture (250 ml) des bactéries transformées suivie d'une extraction de l'ADN par une trousse Maxiprep "QIAGEN" (Chatsworth, CA).

Les mutants de LAG-3 possédant une mutation ciblée ont été préparés à partir d'amorces oligonucléotidiques recouvrant la séquence comprenant le site de la mutation recherchée. Les produits ont été obtenus par PCR (14) ou par insertion d'un oligonucléotide double brin synthétique muté. Le séquençage des produits de PCR a été réalisé avec le kit Sequenase selon les recommandations du fabricant (United State Biochemical Corp., Cleveland, OH).

Les cellules Cos ont été cultivées dans du RPMI 1640 supplémenté avec 10 % de sérum de veau foetal (FCS). Les cellules Cos-7 ont été transfectées avec le plasmide pCDM8-LAG-3C (2) par électroporation en utiisant un appareil Cellject (Eurogentec, Liège, Belgique). L'électroporation a été réalisée dans 500 $\mu$l à 200 v, 1 500 $\mu$F et 30 $\mu$g/ml de plasmide dans un milieu de culture à $5.10^6$ cellules/ml. En bref, $2.10^8$ cellules B ont été incubées pendant 45 minutes à 37°C avec 2 mCi de $^{51}$Cr (volume final = 3 ml). Les cellules ont été lavées trois fois et ensuite utilisées pour les expériences de liaison. Deux jours après la transfection, les cellules Cos ont été traitées avec du PBS 1X EDTA 1 mM et redéposées sur des boites à $0,05.10^6$ cellules/puits dans des plaques pour culture de tissu de 12 puits à fond plat. 24 heures plus tard, $5,5.10^6$ cellules B Daudi marquées au $^{51}$Cr ont été incubées en duplicata dans des puits sur ces monocouches de cellules Cos (volume final = 550 $\mu$l) pendant une heure. Les cellules Daudi ont ensuite été aspirées et les puits ont été lavés cinq à sept fois en ajoutant délicatement goutte à goutte 1 ml de milieu. Les sommets des puits ont été lavés par aspiration avec une pipette Pasteur. Les cellules restantes ont été lysées dans 1 ml de PBS, 1 % Triton pendant 15 minutes à 37°C. Les lysats ont été ensuite centrifugés à 10 000 tours/min pendant 10 minutes et on a effectué le comptage de la radioactivité sur 500 $\mu$l du surnageant résultant. Le nombre de rosettes (plus de 5 cellules Daudi) obtenues avec les mutants LAG-3 a été déterminé par rapport au nombre de rosettes obtenues avec des cellules Cos-7 transfectées avec LAG-3 sauvage. Les cellules Cos-7 transfectées avec le vecteur pCDM8 contenant l'insert LAG-3 dans l'orientation inverse (GAL) servait de contrôle négatif pour la numération de la formation de rosettes.

. Marquage par immunofluorescence et cytométrie de flux des cellules transfectées.

Le niveau d'expression de LAG-3 mutant a été étudié avec la réactivité de l'anticorps 15A9 car l'épitope reconnu par celui-ci (LAG-3.3) n'a jamais été affecté par les mutations. Le niveau d'expression a été calculé au moyen d'unités de fluorescence arbitraire (FU) défini par la formule suivante :

$$FU = \% \text{ de cellule positive x fluorescence moyenne de cellules positives}$$

Pour comparer le niveau d'expression de LAG-3 entre les mutants et la protéine sauvage (Wild-type" ou wt), les niveaux d'expression relatifs ont été calculés comme indiqué ci-après, en utilisant l'anticorps monoclonal 15A9 et un anticorps monoclonal de contrôle (dénommé "nég") :

$$(FU_{15A9}\text{mutant} - FU_{neg}\text{mutant}) \: / \: (FU_{15A9}\text{mutant}) - FU_{neg}\text{wt})$$

La réactivité de l'anticorps monoclonal envers les molécules LAG-3 mutées a été calculée comme ci-après :

$$(FU_{mAb}mutant - FU_{neg}mutant) \, / \, (FU_{15A9}mutant) - FU_{neg}mutant)$$

$$(FU_{mAb}wt - FU_{neg}wt) \, / \, (FU_{15A9}wt) - FU_{neg}wt)$$

. Production et purification de LAG-3D1D2Ig et LAG-3D1D4Ig

Les cellules Cos ont été cultivées dans du milieu de RPMI-1640 supplémenté avec 10 % de sérum de veau foetal (FCS). Environ $10^7$ cellules Cos ont été transfectées avec 50 μg soit de pCMD7-LAG-3D1D2Ig soit de pCDM7-LAG-3D1D4Ig par électroporation en utilisant un appareil Cellject (Eurogentec, Liège, Belgique).

Les cellules transfectées ont été ensemencées sur des boites de Petri avec du milieu RPMI contenant 5 % de FCS à une densité de $2,5.10^5$ cellules par ml. Douze heures plus tard, le milieu de culture a été ôté et remplacé par du milieu RPMI sans FCS. Les surnageants de culture ont été récoltés et de nouveau remplacés par du milieu RPMI frais sans FCS aux jours 3, 6, 9 et 12 après transfection. Les surnageants de culture ont ensuite été centrifugés à basse vitesse pour éliminer les débris cellulaires, puis clarifiés par centrifugation à 10 000 Xg pendant 30 minutes et filtrés à travers des filtres d'acétate de cellulose de 0,22 μ.

Les immunoadhésines ont alors été purifiées par chromatograpie d'affinité en utilisant une colonne de protéine A Sépharose (Pharmacia, Suède) équilibrée avec du citrate de sodium 0,05 M pH8. Après adsorption des immunoadhésines, la colonne a été lavée avec du tampon citrate de sodium 0,05 M, pH8 et les protéines spécifiquement retenues par la colonne ont été éluées avec un tampon citrate de sodium 0,05 M pH3. Afin d'éviter une détérioration des immunoadhésines, toutes les fractions ont été neutralisées avec du Tris HCl 2M pH8 puis dialysées contre du milieu RPMI. L'analyse par SDS-PAGE suivie d'une coloration au nitrate d'argent a permis de déterminer que les immunoadhésines ont été purifiées à homogénéité. Après ultrafiltration sur membranes de polyestersulfones (Filtron, Northborough, MA), l'analyse de la liaison de ces immunoadhésines sur des cellules Daudi MHC classe II positives par immunofluorescence a pu être directement réalisée.

**EXEMPLES**

I - Construction des mutants LAG-3 : R73E, R75A, R75E et R76E

Le fragment Eco47III-BgIII de 1620 paires de bases appelé FDC (Triebel et al., 1990) codant pour la molécule LAG-3 entière, a été introduit après addition de segments de liaison ("linkers") BstXI (Invitrogen, San Diego, CA) dans le vecteur d'expression pCDM8 lui-même linéarisé par digestion avec l'enzyme BstXI (figure 1). Les plasmides contenant le gène LAG-3 sous le contrôle du promoteur du cytomégalovirus ou le gène LAG-3 dans l'orientation inverse ont été sélectionnés et dénommés pCDM8-LAG-3c et pCDM8-GAL, respectivement.

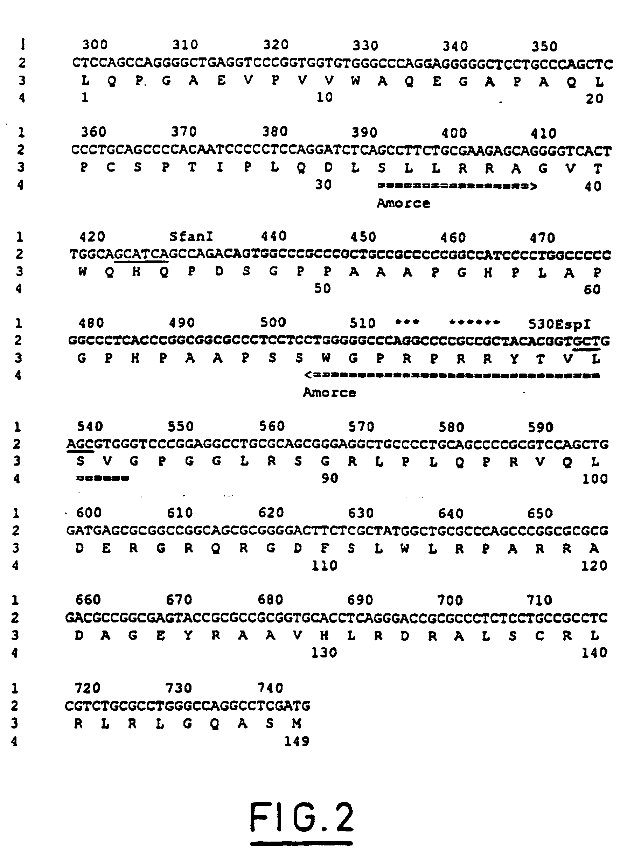

Les fragments d'ADN portant des mutations ponctuelles aux codons des acides aminés 73, 75 et 76 ont été obtenus par amplification par PCR en utilisant l'amorce dénommée OU (brin codant) dans le tableau 1 et l'une des amorces dénommée R73E, R75A, R75E ou R76E (brin complémentaire) dans le tableau 1. Les fragments d'ADN contenant la mutation désirée ont ensuite été digérés par les enzymes de restriction EspI et SfanI et les différents fragments mutés EspI-SfanI de 103 paires de bases (figures 2 et 3) ont été purifiés après électrophorèse en gel d'agarose.

Parallèlement, pCDM8-LAG-3 a été digéré soit par XhoI et SfanI soit par EspI et NotI. Le fragment XhoI-SfanI de 246 paires de bases contenant la partie 5' de l'ADNc de LAG-3, le fragment EspI-XhoI de 1362 paires de bases contenant la partie 3' de l'ADNc de LAG-3 et le fragment XhoI-NotI de 3 900 paires de bases contenant pCDM7 ont été purifiés et liés avec l'un des quatre fragments mutés EspI-SfanI de 103 paires de bases.

Les plasmides portant les séquences de chaque forme mutée ont été sélectionnés. Le séquençage nucléotidique des séquences amplifiées par PCR de chaque forme mutée a confirmé la présence des mutations ponctuelles dans les codons des acides aminés 73, 75 et 76.

Tableau 1

| Amorces | Séquences |
|---|---|
| Amorce OU | 5' GCC TCC TGC GAA GAG CAG GGG T 3' |
| Position<br>Séquence de LAG-3<br>Brin codant<br>Brin complémentaire | 82  81  80  79  78  77  76  75  74  73  72  71  70<br>Val Ser Leu Val Thr Tyr Arg Arg Pro Arg Pro Gly Trp<br>3' GTG CGA GTC GTG GCA CAT CGC CGC CCC GGA CCC GGG GGT 5'<br>5' CAC GCT CAG CAC CGT GTA GCG GCG GGG CCT GGG CCC CCA 3' |
| Amorce R73E |     Glu<br>3'         GAG     5'<br>5' C CAC GCT CAG CAC CGT GTA GCG GCG GGG <u>CTC</u> GGG CCC CCA 3' |
| Amorce R75A |     Ala<br>3'         CCG     5'<br>5' C CAC GCT CAG CAC CGT GTA GCG <u>GGC</u> GGG CCT 3' |
| Amorce R75E |     Glu<br>3'         AAG     5'<br>5' C CAC GCT CAG CAC CGT GTA GCG <u>TTC</u> GGG CCT 3' |
| Amorce R76E |     Glu<br>3'         AAG     5'<br>5' C CAC GCT CAG CAC CGT GTA <u>TTC</u> GCG GGG CCT 3' |

Le tableau 1 représente une séquence ; nucléotidique 5'→ 3' des amorces utilisées pour introduire des mutations ponctuelles aux codons 73, 75 et 76.

Les codons mutés sont soulignés. Les séquences des codons sur le brin complémentaire (sens 3'→5') et les nouveaux acides aminés résultant de ces modifications sont indiqués.

La séquence en acides aminés de la séquence naturelle de LAG-3 et leur position respective sont indiquées à titre de référence.

Des approches similaires bien connues de l'homme du métier ont été utilisées pour générer les autres formes mutées de LAG-3 dans le tableau 2.

II - Analyse des mutants LAG-3 dans un test d'adhésion cellulaire

Les résidus auxquels il est fait référence sont numérotés selon la séquence N-terminale de la protéine mature. Les substitutions indiquées ont été réalisées afin de recouvrir les régions telles que la boucle externe, le domaine CDR1, 2 et 3. Des substitutions unitaires d'acides aminés ont été réalisées dans la molécule LAG-3 (excepté en ce qui concerne le double-mutant D133A/R134A et le mutant de délétion de la boucle externe 54/66) par mutagénèse dirigée. En général, l'acide aminé substitué était remplacé par l'alanine. D'autres substitutions ont été réalisées afin de préserver ou modifier l'hydrophobicité, la fonction de liaison de l'hydrogène ou la charge.

Les mutants obtenus ont été analysés dans un test d'adhésion cellulaire. Dans ce système, les formes mutantes et sauvages de LAG-3 sont exprimées dans des cellules de singe Cos-7 transformées par le virus simien SV40 et sont testées quant à leur capacité de se lier aux cellules Daudi exprimant les molécules de classe II (2). Comme le niveau de l'expression à la surface cellulaire variait d'une expérience à l'autre avec cet essai, l'expression de chaque mutant a été déterminée et comparée avec l'expression de LAG-3 sauvage dans chacune des expériences. Dans la plupart des cas, l'expression à la surface et l'intégrité structurale de chaque protéine mutante a été confirmée par analyse en fluorescence de cellules activées (FACS) en utilisant les anticorps monoclonaux 17B4 11E3 et 15A9 (tableau 2). En réalité, la plupart des mutations ne produisait pas d'effet au niveau de la liaison de l'anticorps monoclonal. La délétion de la pointe de la boucle externe (54/66) détruisait partiellement ou complétement les épitopes LAG-3.2 (11E3) et LAG-3.1(17B4) respectivement. Les derniers épitopes sont formés en partie par les résidus R73 et H56 respectivement comme le démontre l'absence de réactivité de mutants ponctuels LAG-3 R73E, H56F et H56A avec l'anticorps monoclonal correspondant. Etant donné que la réduction ou la perte de liaison de l'anticorps est limitée à un seul anticorps monoclonal, cet effet a été interprété comme étant une conséquence directe de la mutation dans un résidu exposé et non comme le résultat d'une altération structurale majeure dans la molécule.

La capacité de chaque mutant LAG-3 à se lier aux cellules Daudi classe II[+] a été déterminée dans un test d'adhésion cellulaire quantitatif (voir tableau 2). Après addition des cellules Daudi aux cellules Cos transfectées avec LAG-3 sauvage, des agrégats caractéristiques ont été formés (2) et la formation de rosettes a été analysée au microscope.

La liaison de cellules B après transfection de LAG-3 était restreinte aux cellules Cos-7/LAG-3$^+$ exclusivement comme cela pouvait être observé après fixation et coloration à l'immunoperoxydase avec un anticorps monoclonal anti-LAG-3. Par ailleurs, le niveau de l'expression des mutants de LAG-3 a été déterminé dans chaque expérience par immunofluorescence avec l'anticorps monoclonal 15A9 (l'épitope LAG-3.3 n'était altéré par aucune mutation). Approximativement, 50 % des cellules transfectées avec l'ADNc de LAG-3 mutant ou sauvage exprimaient la molécule dans chaque expérience individuelle. Dans la plupart des cas, les molécules de LAG-3 mutant était exprimées à des niveaux similaires à celui de la molécule sauvage et donc, la liaison des cellules Daudi à ces mutants pouvait être directement comparée avec la liaison à LAG-3 sauvage. La liaison des cellules Daudi aux mutants LAG-3 a été analysée quantitativement avec des lymphocytes B marqués au $^{51}$Cr dans trois expériences indépendantes. Les résultats de l'une de ces expériences sont indiqués dans la figure 4. Les mutants R103A et R88A se lient faiblement aux cellules Daudi. Au contraire, le remplacement à la position 75 d'un acide aminé chargé positivement par de l'alanine (R75A) augmente fortement la liaison.

EP 0 942 973 B1

Tableau 2 : Effet de différentes mutations de LAG-3 sur l'interaction entre LAG-3 et les molécules du CMH de classe II

| Mutation | Localisation du résidu | Anticorps monoclonal anti-LAG-3 | | | Niveau d'expression de LAG-3 | Adhésion |
|---|---|---|---|---|---|---|
| | | 17B4 | 11E3 | 15A9 | | |
| wt (sauvage) | - | + | + | + | 1,0 | 1 |
| GAL | témoin négatif | - | - | - | 0 | 0,09 ± 0,06 |
| Q13A | AB | + | + | + | 1,0 | 0,92 ± 0,06 |
| D30A | BC | + | + | + | 0,81 | 0,41 ± 0,17 |
| H56A | boucle externe | - | + | + | 0,7 | 0,45 ± 0,28 |
| H56F | boucle externe | - | + | + | 0,76 | 0,89 ± 0,36 |
| H63A | boucle externe | + | + | + | 0,89 | 0,83 ± 0,10 |
| H63F | boucle externe | + | + | + | 0,95 | 0,91 ± 0,18 |
| R73E | boucle externe | + | - | + | 1,1 | 3,25 ± 1,00 |
| R75A | boucle externe | + | + | + | 0,67 | 4,20 ± 0,66 |
| R75E | boucle externe | + | + | + | 1,4 | 4,10 ± 0,90 |
| R76E | boucle externe | + | + | + | 0,85 | 2,95 ± 1,00 |
| Y77F | C" | + | + | + | 0,75 | 0,05 ± 0,06 |
| R88A | C"D | + | + | + | 0,82 | 0,18 ± 0,07 |
| R103A | DE | + | + | + | 0,93 | 0,49 ± 0,03 |
| R107K | E | + | + | + | 1,1 | 1,02 ± 0,19 |
| D109E | E | + | + | + | 0,81 | 0,18 ± 0,19 |
| R115A | EF | + | + | + | 0,74 | 0,23 ± 0,05 |
| D133A/R134A | FG | + | + | + | 0,99 | 0,78 ± 0,24 |
| D225L | FG(D2) | + | + | + | 0,85 | 0,89 ± 0,13 |
| (D2)⁻ | délétion de D2 | + | + | + | 0,6 | 0,11 ± 0,09 |
| (54/66)⁻ | délétion de la | - | ± | + | 0,6 | 0,08 ± 0,12 |

Le tableau 2 indique quel est le taux de liaison de cellules B Daudi marquées au $^{51}$Cr ou à des cellules Cos-7 exprimant des mutants de LAG-3. Toutes les mutations ont été induites dans le domaine 1, excepté en ce qui concerne D225L. La liaison non spécifique à des cellules témoins transfectées n'a pas été soustraite des cpm moyens expérimentaux et les résultats exprimés par rapport à ceux obtenus avec LAG-3 sauvage doivent donc être comparés au bruit de fond défini par les cellules témoins transfectées par GAL (insert de LAG-3 cloné en orientation inverse dans pCDM8). Le niveau d'expression des mutants de LAG-3 a été comparé à celui de LAG-3 sauvage. Il a été étudié par mesure de la réactivité de l'anticorps monoclonal 15A9. La réactivité de l'anticorps monoclonal relative a été déterminée comme ci-après : + : 1-0,6 ; +/-:0,2-0,6 ; - : 0-0,2.

III - La diminution de la liaison aux molécules de classe II n'est pas le résultat d'une expression de surface réduite des mutants LAG-3.

Les molécules de mutants LAG-3 pour lesquelles les niveaux d'expression étaient similaires à celui de LAG-3 sauvage ont été testées quant à leur capacité de se lier aux cellules Daudi exprimant les molécules de classe II. La formation de rosettes et les coups par minute ont été évalués et comparés aux valeurs obtenues avec LAG-3 sauvage. Toutefois, les mutants D30A, H56A, Y77F, R115A et les mutants de délétion (D2)- et (54/66)- étaient exprimés à des niveaux plus faibles que la molécule LAG-3 sauvage. Ceux-ci ont donc été réexaminés dans des expériences qui ont permis d'écarter la possibilité selon laquelle les effets sur la liaison aux molécules de classe II du CMH pouvaient être dus à une différence dans le niveau d'expression des molécules LAG-3. Dans ces expériences (voir par exemple figure 5), l'expression de LAG-3 sauvage a été modulée en utilisant des quantités variables d'ADN dans les transfections, afin de recouvrir les différents niveaux d'expression des LAG-3 mutants. Pour chacun des six mutants mentionnés ci-dessus les cpm liés étaient inférieurs à 40 % de ceux obtenus avec les cellules exprimant LAG-3 sauvage à des taux d'expression comparables. Ces valeurs brutes devaient toutefois être comparées au bruit de fond, qui, dans cette expérience était de 1048 CPM pour les cellules Cos transfectées par GAL.

Comme l'indique la figure 5, une fonction de liaison diminuée est donc le résultat d'une substitution en acides aminés ou de la délétion d'un fragment et non pas la conséquence d'une expression de surface réduite.

IV - Effet de changement à un site unique sur les domaines de surface 1 et 2

Les effets de mutations dans la structure de LAG-3 sur sa liaison aux molécules de classe II n'ont jamais été rapportés auparavant. De façon inattendue, certaines mutations étaient capables de diminuer la liaison aux molécules de classe II alors que d'autres mutations augmentaient l'affinité de LAG-3 pour les molécules de classe II (tableau 2). Les mutants Y77F, R88A, D109E et R115A avaient une liaison fortement inhibée alors que celles des mutants D30A, H56A et R103A était diminuée uniquement d'un facteur 2. Au contraire, les mutants R73E, R75A, R75E et R76E avaient une liaison augmentée d'un facteur 3 ou plus. On notera que tous ces résidus sont localisés dans la boucle externe, ce qui suggère un rôle important de cette région dans les interactions avec les molécules de classe II.

Un autre argument en faveur de l'implication potentielle de la boucle externe de LAG-3 dans la liaison aux molécules de classe II est fourni par l'analyse du mutant H56A pour lequel la liaison est diminuée d'au moins deux fois (0,45 $\pm$ 0,28 pour un contrôle négatif GAL de 0.09 $\pm$ 0,06) alors qu'une substitution par un acide aminé similaire (H56F) ne donne pas lieu à une altération de la liaison. Le résidu H56, qui fait partie de l'épitope 17B4 (voir tableau 2), est donc supposé être en contact direct avec la molécule de classe II. Cette hypothèse est également supportée par le fait que le mutant de délétion (54/66)⁻ qui est dépourvu du sommet de la boucle externe, ne se lie pas aux molécules de classe II.

V - Une forme tronquée de la protéine soluble révèle un rôle critique des domaines 1 et 2 de LAG-3 dans la liaison aux molécules du CMH de classe II.

Pour évaluer si les deux premiers domaines de LAG-3 contenaient un site de liaison aux molécules de classe II, les auteurs ont synthétisé des protéines de fusion constituées d'un fragment Fc d'IgG1 humaine et des domaines extracellulaires D1 et D2 de LAG-3 (dénommée LAG-3D1D2Ig) ou des quatre domaines extracellulaires de LAG-3 (LAG-3D1D4Ig).

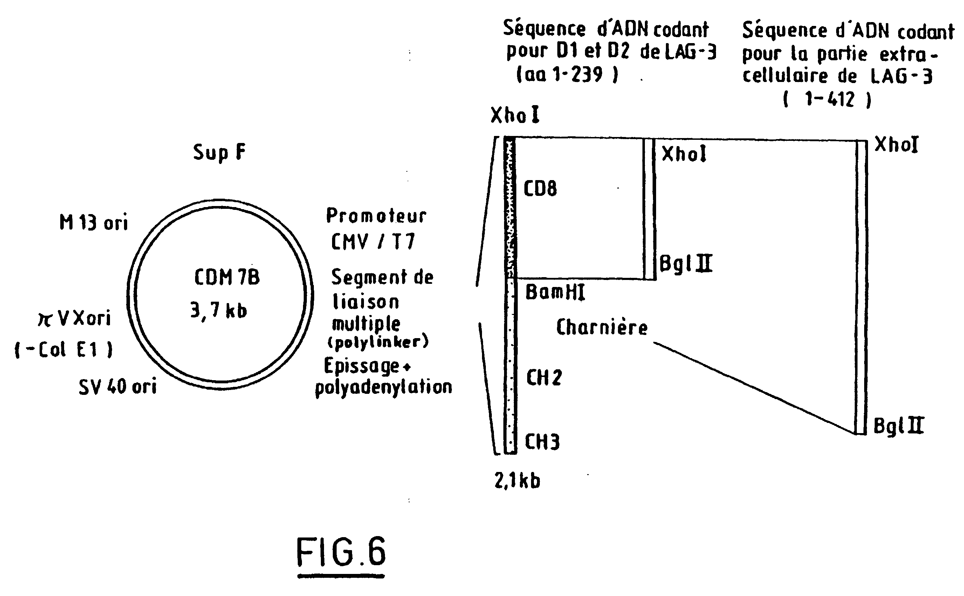

Pour ce faire, un fragment d'ADN de 2,1 kb contenant les séquences codant pour la partie extracellulaire de CD8 fusionnées à la partie Fc d'une immunoglobuline humaine d'isotype IgG1 (soit les régions CH2 et CH3 et la région charnière) a été introduit dans pCDM7 pour donner le plasmide pCDM7-CD8IgG1 (figure 6).

Un fragment d'ADN codant pour le domaine extracellulaire de LAG-3 (4 domaines) a été cloné par la technique de "Hot Start" PCR (trousse AmpliWax PCR Gem 100 de Perkin Elmer Cétus) grâce à l'utilisation des deux amorces nucléotidiques comportant des sites de restriction :

**1) 5'GCCGCCTCGAGGCCCAGACCATAGGAGAGATGT3'**

comprenant le site de restriction XhoI (souligné en trait pointillé) et une courte séquence 5' non codante suivies du codon d'initiation de la traduction du gène LAG-3 (souligné en trait plein).

**2) 5' GCGCCAGATCT ACC TGG GCT AGA CAG CTC TGA GAA 3'**

(412)                              (405)

comprenant un site de restriction BglII (souligné en trait pointillé) et les codons des résidus 405 à 412 de la molécule LAG-3.

Le fragment d'ADN ainsi amplifié a été digéré par les enzymes XhoI et BglII est inséré dans pCDM7-CD8-IgG1 lui-même digéré par les enzymes BamHI et XhoI, à la place des séquences codant pour la partie extracellulaire de CD8 (Fig. 6). Un gène chimère constitué des séquences des 412 premiers acides aminés de LAG-3 fusionnées aux séquences de la partie Fc d'une IgG1 humaine a ainsi été construit. Ce gène chimère est contenu dans le plasmide dénommé pCDM7LAG-3D1D4Ig sous le contrôle du promoteur du cytomégalovirus. La séquence du gène chimère a été vérifiée par séquençage nucléotidique.

Le fragment d'ADN codant pour les deux premiers domaines de LAG-3 a été amplifié et inséré dans pCDM7 CD8-IgG1 comme décrit ci-dessus, excepté que la seconde amorce avait pour séquence :

```
5' GCGCAGATCT ACC CAG AAC AGT GAG GTT ATA CAT 3'
          ------
   Bgl II  (239)                        (232)
```

incluant un site de restriction BglII (souligné en trait pointillé) et les codons des acides aminés 232 à 239. Le plasmide résultant de cette recombinaison,pCDM7-LAG-3D1D2Ig, contient la séquence de LAG-3 codant pour les 239 premiers acides aminés fusionnée à la séquence codant pour la partie Fc d'une IgG (figure 6).

Les molécules de fusion LAG-3D1D2Ig et LAG-3D1D4Ig ont été produites par expression transitoire dans des cellules Cos et purifiées par chromatographie d'affinité sur colonne de protéine A Sépharose.

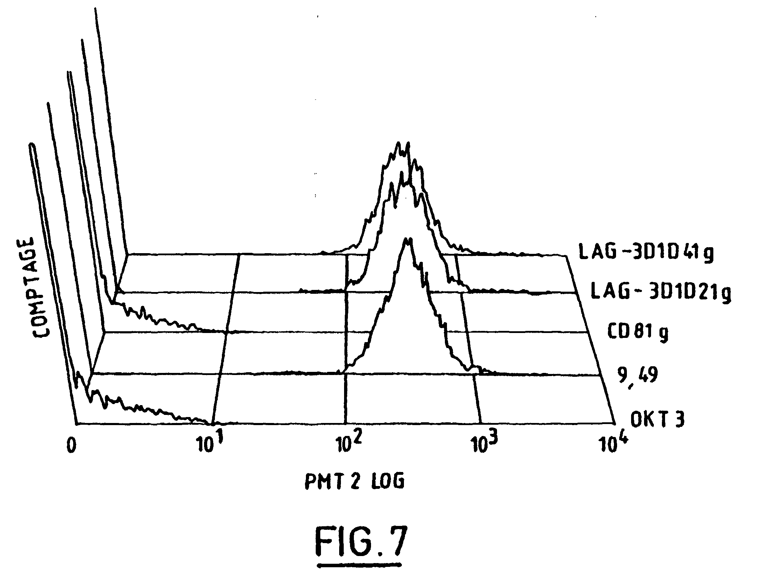

La liaison de LAG-3D1D2Ig à des cellules Daudi classe II[+] était aussi intense que celle de LAG-3D1D4Ig ou d'un anticorps monoclonal anticlasse II (voir figure 7). En conséquence, la structure codée par l'exon 2, 3 et 4 (domaines "Ig-like" 1 + 2) est suffisante pour que la liaison aux molécules de classe II se produise. De plus, la délétion de l'exon 4 (domaine "Ig-like" D2) provoque une inhibition de la formation de rosettes entre cellules Daudi et transfectants Cos-7 (voir tableau 1), malgré une expression de surface correcte sur les cellules Cos-7 des épitopes exprimés sur D1. Ces données suggèrent que le domaine D2 est impliqué soit dans la liaison LAG-3 molécule de classe II, soit dans le positionnement de D1 lors de la liaison avec les molécules de classe II.

Ces résultats permettent également de conclure que les mutants de LAG-3 selon l'invention peuvent avantageusement correspondre à une forme mutée de la forme tronquée de LAG-3 soluble correspondant aux domaines D1 et D2.

VI - Mise en évidence de deux types de mutants négatifs

Dans cette expérience, les auteurs de la présente invention ont montré que trois des mutants négatifs semblaient intervenir dans l'oligomérisation des molécules LAG-3 plutôt que dans l'interaction directe avec les molécules de classe II, le résultat final restant le même (diminution du nombre de rosettes) car l'oligomérisation de LAG-3 paraît essentielle pour stabiliser la liaison aux molécules du CMH de classe II. Dans cette expérience, les auteurs de l'invention sont partis de l'hypothèse selon laquelle une oligomérisation des molécules LAG-3 sur la surface cellulaire pourrait accroître l'interaction entre LAG-3 et les molécules de classe II conduisant ainsi à une stabilisation du complexe. Ceux-ci ont donc admis que si des oligomères de LAG-3 étaient nécessaires pour stabiliser la liaison aux molécules de classe II, certaines mutations de LAG-3 induisant chacune individuellement une diminution de la liaison aux molécules de classe II, pourraient avoir également un effet inhibiteur sur la capacité de molécules de LAG-3 sauvage co-transfecté de se lier aux molécules de classe II, étant donné que la molécule sauvage et la molécule mutante seraient incorporées dans les mêmes oligomères.

Les auteurs de l'invention ont trouvé trois mutations négatives dominantes de LAG-3, R88A, D109E et R115A situées sur une face latérale de la molécule (brin βABED) qui non seulement préviennent la fixation aux molécules du CMH de classe II, mais inhibent également la liaison de la protéine LAG-3 sauvage à ces mêmes molécules (figure 8a). Cette inhibition est dépendante de la quantité d'ADN LAG-3 mutant transfecté (figure 8b). D'autres mutations négatives au contraire, à savoir les mutations Y77F et R103A, affectant des résidus localisés sur le sommet du domaine D1 de LAG-3 n'ont pas démontré le même effet inhibiteur de la liaison de la molécule sauvage aux molécules du CMH de classe II (figure 8a) ; et le nombre de cpm était d'ailleurs plus important pour (Y77F + wt) et (Y103A + wt) que pour wt seul, étant donné que ces protéines mutantes exprimées sont capables de se lier, même faiblement, aux molécules du CMH de classe II.

Dans ces expériences de transfection, le rapport d'ADN mutant sur ADN sauvage utilisé était de 4/1.

**REFERENCES**

1. Triebel, F., S. Jitsukawa. E. Baixeras, S. Roman-Roman, C. Genevee, E. Viegas-Pequignot, and T. Hercend. 1990. LAG-3, a novel lymphocyte activation gene closely related to CD4. *J. Exp. Med* 171:1393.

2. Baixeras. E., B. Huard, C. Miossec, S. Jitsukawa, M. Martin, T. Hercend, C. Auffray, F. Triebel, and D. Piatier-Tonneau. 1992. Characterization of the lymphocyte activation gene 3-encoded protein. A new ligand for human leukocyte antigen class II antigens. *J Exp. Med* 176:327.

3. Huard, B., P. Prigent, F. Pages, D. Bruniquel, N. Borie, and F. Triebel. 1995. T cell MHC class II molecules downregulate CD4+ T cell response following LAG-3 binding. *Journal of immunology (in press)*.

4. Huard. B., P. Prigent, M. Tournier, D. Bruniquel, and F. Triebel. 1995. CD4/major histocompatibility complex class II Interaction analyzed with CD4- and lymphocyte activation gene-3 (LAG-3)-Ig fusion proteins. *Eur. J. immunol.* 25: 2718.

5. Huard. B., M. Tournier, T. Hercend, F. Triebel, and F. Faure. 1994. Lymphocyte-activation gene 3/major histocompatibility complex class II interaction modulates the antilgenic response of CD4+ T lymphocytes. *Eur. J. Immunol.* 24:3216.

6. Huard, B., P. Prigent, F. Pages, D. Bruniquel, and F. Triebel. 1996. T cell MHC class II molecules downregulate CD4+ T cell clone response following LAG-3 binding. *European Journal of Immunology* (sous presse).

7. Gribben, J.G., G.J. Freeman, V.A. Boussiotis, P. Rennert, C.L Jellis, E. Greenfield, M. Barber, V.A. Restivo, X. Ke, G.S. Gray, and L.M. Nadler. 1995. CTLA4-mediates antigen-specific apoptosis of human T cells. *Proc. Natl. Acad. Sci. USA* 92:811.

8. Sun, J., D.J. Leahy, and P.B. Kavathas. 1995. Interaction between CD8 and major histocompatibility complex (MHC) class I mediated by multiple contact surfaces that include the alpha2 and alpha3 domains of MHC class I. *The Journal of Experimental Medicine* 182:1275.

9. Nag, B., H.G. Wada, D. Passmore, B.R. Clark, S.D. Sharma, and H.M. McConnell. 1993. Purified beta-chain of MHC class II binds to CD4 molecules on transfected HeLa cells. *J. Immunol* 150:1358.

10. Konig, R., X. Shen, and R.N. Germain. 1995. Involvement of both major histocompatibility complex class II alpha and beta chains in CD4 function indicates a role for ordered oligomerization in T cell activation. *The Journal of Experimental Medicine* 182.

11. Giblin, P.A., D.J. Leahy, J. Mennone, and P.B. Kavathas, 1994. The role of charge and multiple faces of the CD8alpha/alpha homodimer in binding to major histocompatibility complex class I molecules : support for a bivalent model. *Proc. Natl. Acad. Sci. USA* 91:1716.

12. Fleury, S., D. Lamarre, S. Meloche, S.E. Ryu, C. Candn, W.A. Hendrickson, and R.P. Sekaly. 1991. Mutational analysis of the interaction between CD4 and class II MHC : class II antigens contact CD4 on a surface opposite the gp120-binding site. *Cell* 66:1037.

13. Moebius, U., P. Pallal, S.C. Harrison, and E.L. Reinherz. 1993. Delineation of an extended surface contact area on human CD4 involved in class II major histocompatibility complex binding. *Proc. Natl. Acad. Sci.* USA 90:8259.

14. Miksellan, I., and A. Sergeant. 1992. A general and fast method to generate multiple site directed mutations. *Nucleic Acids Research* 20:376.

**Revendications**

1. Polypeptide purifié correspondant à une forme mutée de la protéine LAG-3 soluble ou à une forme mutée d'un de ses fragments comprenant le domaine extracellulaire D1 et D2 s'étendant des acides aminés 1 à 239 de la protéine LAG-3, ladite mutation consistant :

   - en une substitution d'acide aminé à l'une des positions choisie parmi le groupe constitué par :

     + la position 30 où l'acide aspartique est substitué par l'alanine (D30A),
     + la position 56 où l'histidine est substitué par l'alanine (H56A),
     + la position 77 où la tyrosine est substituée par la phénylalanine (Y77F),
     + la position 88 où l'arginine est substituée par l'alanine (R88A),
     + la position 103 où l'arginine est substituée par l'alanine (RI 03A),
     + la position 109 où l'acide aspartique est substitué par l'acide glutamique (D109E),
     + la position 115 où l'arginine est substituée par l'alanine (RI1SA); ou

   - en une délétion de la région comprise entre la position 54 (P) et la position 66 (A).

2. Séquence nucléotidique isolée codant pour l'un des polypeptides selon la revendication 1, ou sa séquence nucléotidique complémentaire.

3. Polypeptide selon la revendication 1, **caractérisé en ce qu'**il s'agit d'une forme mutée d'un fragment de LAG-3 selon la revendication 1, composé des deux domaines extracellulaires D1 et D2 s'étendant des acides aminés 1 à 239 de la protéine LAG-3 soluble.

4. Polypeptide selon la revendication 1, **caractérisé en ce que** la substitution d'acide aminé est réalisée par mutagénèse dirigée de la séquence d'acide nucléique codant pour la protéine LAG-3 soluble ou un de ses fragments.

5. Vecteur de clonage et/ou d'expression contenant une séquence d'acide nucléique codant pour l'un des polypeptides selon la revendication 1.

6. Vecteur selon la revendication 5, **caractérisé en ce qu'**il s'agit du plasmide pCDM8.

7. Cellule hôte transfectée par un vecteur selon la revendication 5 ou 6.

8. Cellule hôte transfectée selon la revendication 7, **caractérisée en ce qu'**il s'agit d'une cellule de la lignée Cos-7.

9. Méthode de production d'un polypeptide selon la revendication 1, **caractérisée en ce que** l'on cultive des cellules transfectées selon la revendication 7 dans des conditions permettant l'expression d'un polypeptide recombinant selon la revendication 1 et que l'on récupère ledit polypeptide recombinant.

10. Utilisation d'un polypeptide selon la revendication 1 pour la fabrication d'une composition pharmaceutique destinée au traitement des maladies auto-immunes, du rejet de greffe d'organe ou pour une immunothérapie anti-cancéreuse.

11. Composition pharmaceutique comprenant à titre de principe actif un polypeptide selon la revendication 1.

12. Polypeptide selon la revendication 1, **caractérisé en ce qu'**il s'agit d'un polypeptide recombinant produit sous la forme d'une protéine de fusion.

13. Polypeptide selon la revendication 12, **caractérisé en ce que** la fusion est réalisée avec une région d'une immunoglobuline.

14. Polypeptide selon la revendication 1, **caractérisé en ce qu'**il est lié à une toxine ou un radioisotope.

15. Utilisation d'un polypeptide selon la revendication 1 pour la fabrication de composés agonistes ou antagonistes de l'interaction entre LAG-3 et les molécules du CMH de classe II.

**Patentansprüche**

1. Gereinigtes Polypeptid, das einer mutierten Form des löslichen Proteins LAG-3 oder einer mutierten Form eines seiner Fragmente entspricht, die extrazelluläre Domäne D1 und D2 aufweisen, die sich von den Aminosäuren 1 bis 239 des Proteins LAG-3 erstreckt, wobei diese Mutation besteht:

- aus der Ersetzung der Aminosäure an einer Position, die aus der Gruppe ausgewählt wurde, die gebildet wird von:

+ der Position 30, wo die Asparginsäure durch das Alanin (D30A) ersetzt wird,
+ der Position 56, wo das Histidin durch das Alanin (H56A) ersetzt wird,
+ der Position 77, wo das Tyrosin durch das Phenylalanin (Y77F) ersetzt wird,
+ der Position 88, wo das Arginin durch das Alanin (R88A) ersetzt wird,
+ der Position 103, wo das Arginin durch das Alanin (R103A) ersetzt wird,
+ der Position 109, wo die Asparginsäure durch die Glutaminsäure (D109E) ersetzt wird,
+ der Position 115, wo das Arginin durch das Alanin (R115A) ersetzt wird; oder

- aus einer Deletion der Region zwischen der Position 54 (P) und der Position 66 (A).

2. Isolierte Nukleotidsequenz, kodierend für eines der Polypeptide nach Anspruch 1, oder ihre komplementäre Nukleotidsequenz.

3. Polypeptid nach Anspruch 1, **dadurch gekennzeichnet, dass** es sich um eine mutierte Form eines LAG-3-Fragments nach Anspruch 1 handelt, das sich aus den zwei extrazellulären Domänen D1 und D2 zusammensetzt, die sich von den Aminosäuren 1 bis 239 des löslichen Proteins LAG-3 erstrecken.

4. Polypeptid nach Anspruch 1, **dadurch gekennzeichnet, dass** die Ersetzung der Aminosäure durch Mutagenese durchgeführt wird, die von der für das lösliche Protein LAG-3 oder eines seiner Fragmente kodierenden Nukleinsäurensequenz gelenkt wird.

5. Klonierungs- und/oder Expressionsvektor, der eine für eines der Polypeptide nach Anspruch 1 kodierende Nukleinsäurensequenz enthält.

6. Vektor nach Anspruch 5, **dadurch gekennzeichnet, dass** es sich um das Plasmid pCDM8 handelt.

7. Von einem Vektor nach Anspruch 5 oder 6 transfizierte Wirtszelle.

8. Nach Anspruch 7 transfizierte Wirtszelle, **dadurch gekennzeichnet, dass** es sich um eine Zelle der Linie Cos-7 handelt.

9. Methode zur Herstellung eines Polypeptids nach Anspruch 1, **dadurch gekennzeichnet, dass** nach Anspruch 7 transfizierte Zellen hergestellt werden unter Bedingungen, welche die Expression eines rekombinanten Polypeptids nach Anspruch 1 ermöglichen und dass dieses rekombinante Polypeptid wiedergewonnen wird.

10. Verwendung eines Polypeptids nach Anspruch 1 zur Herstellung einer pharmazeutischen Zusammensetzung, die zur Behandlung von Autoimmunerkrankungen, von Organtransplantatabstoßung oder für eine Immunotherapie gegen Krebs bestimmt ist.

11. Pharmazeutische Zusammensetzung, die als Wirkstoffprinzip eine Polypeptid nach Anspruch 1 aufweist.

12. Polypeptid nach Anspruch 1, **dadurch gekennzeichnet, dass** es sich um ein rekombinantes Polypeptid handelt, das in Form eines Fusionsproteins hergestellt wird.

13. Polypeptid nach Anspruch 12, **dadurch gekennzeichnet, dass** die Fusion mit einer Region eines Immunoglobulins durchgeführt wird.

14. Polypeptid nach Anspruch 1, **dadurch gekennzeichnet, dass** es mit einem Toxin oder einem Radioisotop verbunden ist.

15. Verwendung eines Polypeptids nach Anspruch 1 zur Herstellung von agonistischen oder antagonistischen Verbindungen aus der Interaktion von LAG-3 mit den CMH-Molekülen der Klasse II.

**Claims**

1. Purified polypeptide corresponding to a mutated form of the soluble LAG-3 protein or a mutated form of one of its fragments including the extracellular domain D1 and D2 extending from amino acids 1 to 239 of LAG-3 protein, said mutation consisting of:

- a substitution of amino acid at one of the positions chosen from the group consisting of:

+ position 30 at which aspartic acid is substituted by alanine (D30A),
+ position 56 at which histidine is substituted by alanine (H56A),
+ position 77 at which tyrosine is substituted by phenylalanine (Y77F),
+ position 88 at which arginine is substituted by alanine (R88A),
+ position 103 at which arginine is substituted by alanine (R103A),
+ position 109 at which aspartic acid is substituted by glutamic acid (D109E),
+ position 115 at which arginine is substituted by alanine (R115A); or

- deletion of the region between position 54 (P) and position 66 (A).

**2.** Isolated nucleotidic sequence coding for one of the polypeptides according to claim 1, or its complementary nucleotidic sequence.

**3.** Polypeptide according to claim 1, **characterised in that** it is a mutated form of a fragment of LAG-3 according to claim 1, composed of two extracellular domains D1 and D2 extending from amino acids 1 to 239 of the soluble LAG-3 protein.

**4.** Polypeptide according to claim 1, **characterised in that** the amino acid substitution is achieved by site specific mutagenesis of the nucleic acid sequence coding for the soluble LAG-3 protein or one of its fragments.

**5.** Cloning and / or expression vector containing a nucleic acid sequence coding for one of the polypeptides according to claim 1.

**6.** Vector according to claim 5, **characterised in that** it is a pCDM8 plasmide.

**7.** Host cell transfected by a vector according to claim 5 or 6.

**8.** Transfected host cell according to claim 7, **characterised in that** it is a cell in the Cos-7 strain.

**9.** Method of producing a polypeptide according to claim 1, **characterised in that** transfected cells according to claim 7 are cultivated under conditions enabling expression of a recombinant polypeptide according to claim 1 and **in that** said recombinant polypeptide is recovered.

**10.** Use of a polypeptide according to claim 1 to prepare a pharmaceutical composition for the treatment of auto-immune diseases, rejection of the organ graft or for an anti-cancer immunotherapy.

**11.** Pharmaceutical composition comprising a polypeptide according to claim 1 as the active constituent.

**12.** Polypeptide according to claim 1, **characterised in that** it is a recombinant polypeptide produced in the form of a fusion protein.

**13.** Polypeptide according to claim 12, **characterised in that** fusion is achieved with a region of an immunoglobulin.

**14.** Polypeptide according to claim 1, **characterised in that** it is linked to a toxin or a radioisotope.

**15.** Use of a polypeptide according to claim 1 for the production of agonist or antagonist compounds from the interaction between LAG-3 and class II MHC molecules.

FIG.1

```
1       300       310       320       330       340       350
2   CTCCAGCCAGGGGCTGAGGTCCCGGTGGTGTGGGCCCAGGAGGGGGGCTCCTGCCCAGCTC
3     L   Q   P.  G   A   E   V   P   V   V   W   A   Q   E   G   A   P   A   Q   L
4     1                               10                              20

1       360       370       380       390       400       410
2   CCCTGCAGCCCCACAATCCCCCTCCAGGATCTCAGCCTTCTGCGAAGAGCAGGGGTCACT
3     P   C   S   P   T   I   P   L   Q   D   L   S   L   L   R   R   A   G   V   T
4                               30            ==============> 40
                                               Amorce

1       420       SfanI    440       450       460       470
2   TGGCAGCATCAGCCAGACAGTGGCCCGCCCGCTGCCGCCCCCGGCCATCCCCTGGCCCCC
3     W   Q   H   Q   P   D   S   G   P   P   A   A   A   P   G   H   P   L   A   P
4                               50                              60

1       480       490       500       510  ***   ******  S30EspI
2   GGCCCTCACCCGGCGGCGCCCTCCTCCTGGGGGCCCAGGCCCCGCCGCTACACGGTGCTG
3     G   P   H   P   A   A   P   S   S   W   G   P   R   P   R   R   Y   T   V   L
4                               <==================================
                                               Amorce

1       540       550       560       570       580       590
2   AGCGTGGGTCCCGGAGGCCTGCGCAGCGGGAGGCTGCCCCTGCAGCCCCGCGTCCAGCTG
3     S   V   G   P   G   G   L   R   S   G   R   L   P   L   Q   P   R   V   Q   L
4     ======                      90                              100

1      · 600       610       620       630       640       650
2   GATGAGCGCGGCCGGCAGCGCGGGGACTTCTCGCTATGGCTGCGCCCAGCCCGGCGCGCG
3     D   E   R   G   R   Q   R   G   D   F   S   L   W   L   R   P   A   R   R   A
4                               110                             120

1       660       670       680       690       700       710
2   GACGCCGGCGAGTACCGCGCCGCGGTGCACCTCAGGGACCGCGCCCTCTCCTGCCGCCTC
3     D   A   G   E   Y   R   A   A   V   H   L   R   D   R   A   L   S   C   R   L
4                               130                             140

1       720       730       740
2   CGTCTGCGCCTGGGCCAGGCCTCGATG
3     R   L   R   L   G   Q   A   S   M
4                               149
```

# FIG.2

Hind III  Xho I

Sfan 1
Esp I

pCDM 8
LAG-3
5.6 kb

Hind III
Sfan 1
Xho I
Not I

_Hind III

_Xho I + Sfan I    LAG 3 ADNc (fragment Hind III
de 735 pb)

_Esp 1 + Not I

_PCR (amorces sens/inv)

_Sfan 1 + Esp.1

Sfan 1        * Esp 1

103 pb

Xho I        Sfan 1

246 pb

Esp 1  Sfan I  Not I

1360 pb

pCDM 8 Xho-Not I ——— Ligature

3.9 kb

Xho I

Sfan 1
* Esp I

pCDM 8
mutant de
LAG-3
5.6 kb

Sfan 1

Not I

*        Point de mutation

▭▭▭▭  ADNc de LAG-3

———        pCDM 8

# FIG.3

FIG.4

FIG.5

Séquence d'ADN codant pour D1 et D2 de LAG-3 (aa 1-239)

Séquence d'ADN codant pour la partie extra-cellulaire de LAG-3 (1-412)

Sup F

M 13 ori

πV Xori

(-Col E1)

SV 40 ori

CDM 7B

3,7 kb

Promoteur CMV / T7

Segment de liaison multiple (polylinker)

Epissage + polyadenylation

Xho I

Xhol

CD8

Bgl II

BamHI

Charnière

CH2

CH3

2,1kb

Xhol

Xhol

Bgl II

FIG.6

FIG.7

FIG.8